# EUROPÄISCHE PATENTSCHRIFT

(11) **EP 0 304 789 B1**
(45) Veröffentlichungstag und Bekanntmachung des Hinweises auf die Patenterteilung: **17.03.1993**
(21) Anmeldenummer: 88113339.1
(22) Anmeldetag: 17.08.1988
(51) Int. Cl.: C07D 235/26, C07D 235/28, C07D 409/12, C07D 405/12, C07D 403/12, C07D 401/12, C07D 417/12, A61K 31/415

(54) **Benzimidazol-Derivate**
Benzimidazole derivatives
Dérivés de benzimidazole

(30) Priorität: 25.08.1987 DE 3728695
(43) Veröffentlichungstag der Anmeldung: 01.03.1989
(73) Patentinhaber: SCHERING AKTIENGESELLSCHAFT, 13342 Berlin (DE)
(72) Erfinder: Schöllkopf, Klaus, Dr., D-1000 Berlin 38 (DE); Wachtel, Helmut, Dr., D-1000 Berlin 19 (DE)

(56) Entgegenhaltungen:
- EP-A- 0 175 525
- WO-A-86/01204

## Beschreibung

Die erfindungsgemäßen Verbindungen haben die allgemeine Formel I 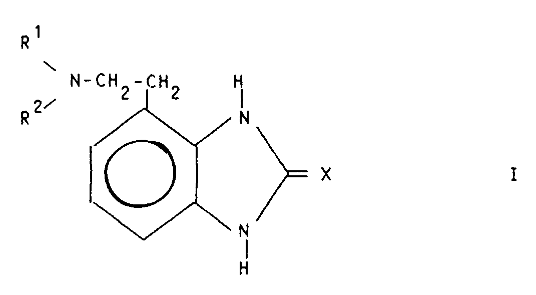
worin
R¹ und R² gleich oder verschieden sind und jeweils Wasserstoff, einen Cycloalkyl- oder Cycloalkylalkylrest mit 3 - 6 Kohlenstoffatomen oder einen Alkylrest mit 1 - 6 Kohlenstoffatomen bedeuten, wobei der Alkylrest ein- oder zweifach mit Phenyl oder einem 5 - oder 6-Ring-Heteroaromaten mit ein bis zwei Schwefel-, Stickstoff- und/oder Sauerstoffatomen substituiert sein kann und der Phenylrest ein- bis dreifach mit C₁₋₄-Alkyl, C₁₋₄-Alkoxy, Hydroxy oder Halogen substituiert sein kann und
X Sauerstoff oder Schwefel ist, sowie deren Säureadditionssalze oder Tautomere.

Die Verbindungen der allgemeinen Formel I beinhalten auch die möglichen tautomeren Formen.

Als Kohlenwasserstoffreste R¹ und R² kommen geradkettige und verzweigte gesättigte niedere Alkylreste mit bis zu 6 Kohlenstoffatomen in Betracht, ferner Cycloalkyl- und Cycloalkyl-alkylgruppen mit 3-6 Kohlenstoffatomen.

Beispielsweise seien die folgenden möglichen Reste genannt: Methyl, Ethyl, Propyl, Isopropyl, Butyl, Isobutyl, tert. Butyl, sek. Butyl, Pentyl, Hexyl, 2-Methylbutyl, 2,2-Dimethylpropyl, Cyclopropyl, Cyclobutyl, Cyclopentyl, Cyclohexyl, Cyclopropylmethyl, Cyclopropylethyl, Cyclopentylmethyl u.a.

Der Alkylrest kann an jeder beliebigen Stelle 1-2-fach substituiert sein mit gegebenenfalls substituierten Aromaten oder Heteroaromaten. Ist der Substituent ein Aromat, so ist der Ar-C₁₋₂-Alkylrest wie der Benzyl- und der Phenethylrest als bevorzugt anzusehen, der gegebenenfalls mit C₁₋₄-Alkyl, C₁₋₄-Alkoxy, Hydroxy oder Halogen, insbesondere Chlor oder Fluor, 1-, 2- oder 3-fach am Aromaten substituiert sein kann. Ist der Substituent ein Heteroaromat, so sind die nachfolgend genannten 5-oder 6-Ring-Heteroaromaten, die ein bis zwei Heteroatome, wie Schwefel, Sauerstoff und/oder Stickstoff enthalten können, geeignet wie zum Beispiel: Thiophen, Furan, Pyrrol, Pyridin, Thiazol, Imidazol, Pyrazol.

Die Salze der erfindungsgemäßen Verbindungen der Formel I sind Säureadditionssalze und leiten sich von den üblicherweise verwendeten anorganischen und organischen Säuren ab, wie z.B. Salzsäure, Bromwasserstoffsäure, Schwefelsäure, Zitronensäure, Maleinsäure oder Fumarsäure.

Benzimidazole können dopaminerge und adrenerge Prozesse beeinflussen, wobei sowohl Wirkungen auf das Zentralnervensystem wie auch periphere Effekte auftreten können (WO-A-8601204, EP-A-175525).

Überraschenderweise zeigte es sich, daß die erfindungsgemäßen Verbindungen in sehr niedrigen Dosen eine höhere dopaminerge Wirkung besitzen als Levodopa oder Bromocriptin bei gleichzeitiger Verminderung von unerwünschten Nebenwirkungen.

Auf Grund ihres Wirkprofils sind die erfindungsgemäßen Verbindungen geeignet zur Behandlung von Krankheiten des zentralen Nervensystems, die durch dopaminerge Wirkstoffe beeinflußt werden wie zum Beispiel Morbus Parkinson, Akromegalie und Hyperprolactinämie, sowie zur Behandlung von Herz-Kreislauf-Erkrankungen wie zum Beispiel Hypertonie, Herzinsuffizienz, Angina pectoris und Durchblutungsstörungen, insbesondere zur Erhöhung des renalen Blutflusses.

Die Herstellung der Verbindungen der allgemeinen Formel I erfolgt nach an sich bekannten Verfahren, indem man beispielsweise die Verbindungen der allgemeinen Formel II 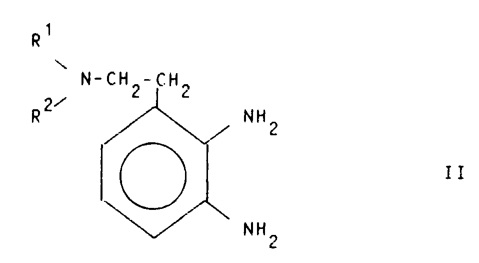
worin R¹ und R² die oben angegebene Bedeutung haben mit einem Kohlensäure- oder Thiokohlensäurederivate cyclisiert und gewünschtenfalls anschließend das Säureadditionssalz bildet.

Als geeignete Kohlensäurederivate seien beispielsweise Phosgen, Harnstoff, Carbonyl-diimidazol, Kohlensäureester und als geeignete Thiokohlensäurederivate beispielsweise Thiophosgen, Thioharnstoff, Thiocarbonyl-diimidazol und Thiokohlensäureester der allgemeinen Formel III R⁴R⁵C = X genannt, worin X Sauerstoff oder Schwefel und R⁴, R⁵ jeweils Chlor, NH₂, Imidazolyl oder C₁₋₄-Alkoxy sind.

Die Cyclisierung wird bei erhöhter Temperatur bis zur Siedetemperatur des Lösungsmittel vorgenommen und ist im allgemeinen nach ca. 3 Stunden beendet.

Als Lösungsmittel sind inerte Lösungsmittel wie Kohlenwasserstoffe, cyclische und acyclische Ether oder aliphatische niedere Alkohole geeignet wie beispielsweise Toluol, Benzol, Xylol, Tetrahydrofuran, Dioxan, Methanol, Ethanol, Propanol.

Zur Bildung von Salzen werden die Verbindungen der allgemeinen Formel I beispielsweise in Alkohol oder Methylenchlorid gelöst und mit einer konzentrierten Lösung der gewünschten Säure in Alkohol bei Raumtemperatur versetzt.

Die Herstellung der Arzneimittelspezialitäten erfolgt (abhängig von der angestrebten Applikationsart: oral, parenteral, intravenös etc.) in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen, Injektionslösungen usw. überführt.

Die Herstellung der Ausgangsverbindungen ist bekannt oder erfolgt nach bekannten Methoden.

Die nachfolgenden Beispiele sollen das erfindungsgemäße Verfahren erläutern.

### Darstellung der Ausgangsverbindungen

### N-[2-(2,3-Diaminophenyl)-ethyl]-N,N-dipropylamin

a) 5.5 g 2,3-Dinitrotoluol werden mit 13.0 g Bis-dipropylamino-tert.-butoxy-methan für zwei Stunden auf 60 °C erhitzt. Nach dem Abkühlen wird das Reaktionsgemisch im Hochvakuum von flüchtigen Bestandteilen befreit. Es werden 7.6 g β-Dipropylamino-2,3-dinitrostyrol als Öl erhalten.
b) 7.6 g der unter a) erhaltenen Verbindungen werden in einem Gemisch aus 80 ml Methanol, 55 ml Tetrahydrofuran und 11 ml Eisessig gelöst und auf -20 °C abgekühlt. Zu dieser Lösung werden 2.1 g Natriumcyanborhydrid portionsweise zugegeben. Anschließend wird zwei Stunden bei 20 °C nachgerührt. Zu der Lösung werden dann 45 ml 2-normale Salzsäure gegeben und es wird eine Stunde nachgerührt. Das Reaktionsgemisch wird daraufhin eingeengt und in Natriumhydrogencarbonatlösung aufgenommen. Die wäßrige Phase wird mehrmals mit Diethylether extrahiert. Die Etherphase wird abgetrennt, mit Natriumsulfat getrocknet und mit Oxalsäure versetzt. Der Niederschlag wird abfiltriert und mit Diethylether nachgewaschen. Anschließend wird mit Natriumhydrogencarbonatlösung die Base freigesetzt und mit Diethylether extrahiert. Nach Entfernen des Lösungsmittels erhält man 1.5 g N-[2-(2,3-Dinitrophenyl)-ethyl]-N,N-dipropylamin als Öl.
c) 0.35 g der unter b) erhaltenen Verbindung werden in 40 ml Methanol gelöst und nach Zugabe von 0.05 g Palladium auf Kohle hydriert. Nach beendeter Wasserstoffaufnahme wird der Katalysator abfiltriert und das Filtrat eingeengt. Es werden 0.3 g N-[2-(2,3-Diaminophenyl)-ethyl]-N,N-dipropylamin als Öl erhalten.

### N-[2-(2,3-Diaminophenyl)-ethyl]-N-(2-phenylethyl)-N-propylamin

a) 9.1 g 2,3-Dinitrotoluol werden mit 15.0 g Bis-dimethylamino tert.-butoxy-methan [Chem. Ber. 101, 41 (1968)] für vier Stunden auf 70 °C erhitzt. Nach dem Abkühlen wird das Reaktionsgemisch im Hochvakuum von flüchtigen Bestandteilen befreit. Es werden 10.1 g β-Dimethylamino-2,3-dinitrostyrol als Öl erhalten.
b) 10.1 g der unter a) erhaltenen Verbindung werden in einer Mischung aus 250 ml Wasser und 100 ml Methanol gelöst. Zu dieser Lösung werden 14.0 g Hydroxylamin-O-sulfonsäure unter Rühren zugegeben. Nach zwölf Stunden wird das Reaktionsgemisch eingeengt und mit Dichlormethan mehrmals extrahiert. Die organische Phase wird über Natriumsulfat getrocknet und abfiltriert. Nach dem Einengen wird der Rückstand aus Ethanol umkristallisiert. Es werden 4.5 g 2,3-Dinitrophenylacetonitril vom Schmelzpunkt 88-90 °C erhalten.
c) 5.4 g der unter b) erhaltenen Verbindung werden in 30 ml 60-prozentiger Schwefelsäure vier Stunden auf 100 °C erhitzt. Nach dem Abkühlen und Abfiltrieren erhält man 4.9 g 2,3-Dinitrophenylessigsäure vom Schmelzpunkt 209-215 °C.
d) Zu einer Lösung von 2.8 g der unter c) erhaltenen Verbindung und 2.0 g N-Propyl-phenethylamin [J. Am. Chem. Soc. 75, 4664 (1953)] in 50 ml Dichlormethan werden bei 0 °C 2.6 g N,N′-Dicyclohexylcarbodiimid, gelöst in 30 ml Dichlormethan, zugegeben. Nach fünfstündigem Rühren bei 20 °C wird abfiltriert. Die Lösung wird mit 2-normaler Salzsäure, 2-normaler Natronlauge und Wasser gewaschen. Nach dem Trocknen über Natriumsulfat wird abfiltriert und eingeengt. Es werden 3.5 g N-Phenylethyl-N-propyl-(2,3-dinitrophenyl)-acetamid als Öl erhalten.
e) 3.4 g der unter d) erhaltenen Verbindung werden in 40 ml Tetrahydrofuran gelöst. Bei 0 °C werden unter Rühren 13 ml einer einmolaren Boranlösung in Tetrahydrofuran zugetropft. Bei 20 °C wird zwölf Stunden nachgerührt. Anschließend wird mit Wasser und dann mit konzentrierter Salzsäure hydrolysiert. Das Reaktionsgemisch wird eingeengt und mit Natriumcarbonatlösung neutralisiert. Es wird mit Ethylacetat extrahiert. Die organische Phase wird über Natriumsulfat getrocknet, abfiltriert und eingeengt. Es werden 2.3 g N-[2-(2,3-Dinitrophenyl)-ethyl]-N-(2-phenylethyl)-N-propylamin als Öl erhalten.
f) 1.5 g der unter e) erhaltenen Verbindung werden in 100 ml Methanol gelöst und nach Zugabe von 0.2 g Palladium auf Kohle hydriert. Nach beendeter Wasserstoffaufnahme wird der Katalysator abfiltriert und das Filtrat eingeengt. Es werden 1.1 g N-[2-(2,3-Diaminophenyl)-ethyl]-N-(2-phenylethyl)-N-propylamin als Öl erhalten.

### N-[2-(2,3-Diaminophenyl)-ethyl]-N-methyl-N-butylamin

a) 9.0 g 2,1,3-Benzothiadiazol-4-essigsäure (Zhurnal Obshchei Khimii 34, 1272 (1964) werden in 100 ml Dichlormethan gelöst und mit 16.8 ml Thionylchlorid versetzt. Das Gemisch wird für fünf Stunden am Rückfluß erhitzt und nach dem Abkühlen werden die flüchtigen Bestandteile im Vakuum entfernt. Der Rückstand wird in 50 ml Dichlormethan aufgenommen und unter Eiskühlung zu einer Lösung von 10.5 ml N-Methyl-butylamin in 50 ml Dichlormethan getropft. Nach einer Stunde wird das Reaktionsgemisch auf Wasser gegeben und die organische Phase wird mit 1-normaler Salzsäure und 1-normaler Natronlauge gewaschen. Die organische Phase wird über Natriumsulfat getrocknet und nach dem Einengen wird der Rückstand bei 0.025 mbar und 170°C destilliert. Es werden 11.2 g N-Butyl-N-methyl-2,1,3-benzothiadiazol-4-acetamid als Öl erhalten.
b) 11.2 g der unter a) erhaltenen Verbindung werden in 100 ml Tetrahydrofuran gelöst. Diese Lösung wird unter einer Stickstoffatmosphäre zu 170 ml einer 1-molaren Boranlösung in Tetrahydrofuran unter Eiskühlung getropft. Das Reaktionsgemisch wird bei 20°C zwanzig Stunden nachgerührt. Anschließend wird vorsichtig mit 100 ml 63-prozentiger Bromwasserstoffsäure versetzt. Das Gemisch wird vier Stunden nachgerührt und im Vakuum weitgehend eingeengt. Der Rückstand wird in Wasser aufgenommen und mit Dichlormethan extrahiert. Die organische Phase wird abgetrennt und eingeengt. Der Rückstand wird aus Aceton umkristallisiert. Es werden 6.3 g 4-[2-(N-Butyl-N-methylamino)-ethyl]-2,1,3-benzothiadiazol Hydrobromid vom Schmelzpunkt 130°C erhalten.
c) 2.0 g der unter b) erhaltenen Verbindung werden in 30 ml Tetrahydrofuran suspendiert. Unter Kühlung werden 0.7 g Lithiumaluminiumhydrid zugegeben. Nach zwei Stunden wird der Ansatz auf Natriumhydrogencarbonatlösung gegeben und mit Diethylether extrahiert. Die organische Phase wird über Natriumsulfat getrocknet und eingeengt. Es werden 1.3 g N-[2-(2,3-Diaminophenyl)-ethyl]-N-methyl-N-butylamin erhalten.

Analog werden hergestellt:

### N-[2-(2,3-Diaminophenyl)-ethyl]-N-[2-(2-thienyl)-ethyl]-N-propylamin

### N-[2-(2,3-Diaminophenyl)-ethyl]-N-[2-(3-thienyl)-ethyl]-N-propylamin

### Beispiel 1

### 4-[2-(N-Butyl-N-methylamino)-ethyl]-2,3-dihydro-2-benzimidazolthion, Hydrobromid

650 mg N-[2-(2,3-Diaminophenyl)-ethyl]-N-methyl-N-butylamin und 600 mg N,N′-Thiocarbonyldiimidazol werden in 30 ml Tetrahydrofuran gelöst und drei Stunden am Rückfluß erhitzt. Nach dem Abkühlen wird weitgehend eingeengt. Der Rückstand wird in Wasser aufgenommen und mit Essigester extrahiert. Die organische Phase wird über Natriumsulfat getrocknet und eingeengt. Der Rückstand wird in Ethanol aufgenommen und mit 33-prozentiger Bromwasserstoffsäure versetzt. Nach erneutem Einengen wird der Rückstand aus Isopropanol umkristallisiert. Es werden 370 mg 4-[2-(N-Butyl-N-methylamino)-ethyl]-2,3-dihydro-2-benzimidazolthion, Hydrobromid vom Schmelzpunkt 264-266°C erhalten.

### Beispiel 2

### 4-[2-[N-(2-Phenylethyl)-N-propylamino]-ethyl]-2,3-dihydro-2-benzimidazolthion, Hydrobromid

Aus 370 mg N-[2-(2,3-Diaminophenyl)-ethyl]-N-(2-phenylethyl)-N-propylamin und 230 mg N,N′-Thiocarbonyldiimidazol werden nach der in Beispiel 1 beschriebenen Weise 170 mg 4-[2-[N-(2-Phenylethyl)-N-propylamino]-ethyl]-2,3-dihydro-2-benzimidazolthion, Hydrobromid vom Schmelzpunkt 198-201°C erhalten.

### Beispiel 3

### 4-[2-[N-Propyl-N-[2-(2-thienyl)-ethylamino]]-ethyl]-2,3-dihydro-2-benzimidazolon, Fumarat

1.40 g N-[2-(2,3-Diaminophenyl)-ethyl]-N-[2-(2-thienyl)-ethyl]-N-propylamin und 0.70 g N,N′-Carbonyldiimidazol werden in 50 ml Tetrahydrofuran gelöst und drei Stunden am Rückfluß erhitzt. Nach dem Abkühlen wird weitgehend eingeengt. Der Rückstand wird in Wasser aufgenommen und mit Essigester extrahiert. Die organische Phase wird über Natriumsulfat getrocknet und eingeengt. Der Rückstand wird in Ethanol aufgenommen und mit Fumarsäure versetzt. Nach erneutem Einengen wird der Rückstand aus Isopropanol umkristallisiert. Es werden 0.55 g 4-[2-[N-Propyl-N-[2-(2-thienyl)-ethylamino]]-ethyl]-2,3-dihydro-2-benzimidazolon, Fumarat vom Schmelzpunkt 170-173°C erhalten.

### Beispiel 4

### 4-[2-(N,N-Dipropylamino)-ethyl]-2,3-dihydro-2-benzimidazolthion, Hydrobromid

360 mg N-[2-(2,3-Diaminophenyl)-ethyl]-N,N-dipropylamin werden mit 30 ml Tetrahydrofuran und 360 mg N,N′-Thiocarbonyldiimidazol drei Stunden am Rückfluß erhitzt. Nach dem Einengen wird der Rückstand mit Wasser und Methanol versetzt. Der Niederschlag wird in Ethanol gelöst und mit Bromwasserstoffsäure in das Hydrobromid überführt. Es werden 120 mg 4-[2-(N,N-Dipropylamino)-ethyl]-2,3-dihydro-2-benzimidazolthion, Hydrobromid vom Schmelzpunkt 250-255 °C erhalten.

### Beispiel 5

### 4-[2-(N,N-Dipropylamino)-ethyl]-2,3-dihydro-2-benzimidazolon, Hydrobromid

360 mg N-[2-(2,3-Diaminophenyl)-ethyl]-N,N-dipropylamin werden mit 30 ml Tetrahydrofuran und 300 mg N,N′-Carbonyldiimidazol drei Stunden am Rückfluß erhitzt. Nach dem Einengen wird der Rückstand in wäßrigem Alkohol aufgenommen und mit Bromwasserstoffsäure versetzt. Nach erneutem Einengen wird der Rückstand aus Isopropanol/Diethylether umkristallisiert. Es werden 90 mg 4-[2-(N,N-Dipropylamino)-ethyl]-2,3-dihydro-2-benzimidazolon, Hydrobromid vom Schmelzpunkt 189 - 192 °C erhalten.

### Beispiel 6

### 4-[2-(N-Butyl-N-methylamino)-ethyl]-2,3-dihydro-2-benzimidazolon, Hydrobromid

Aus 400 mg N-[2-(2,3-Diaminophenyl)-ethyl]-N-methyl-N-butylamin und 350 mg N,N′-Carbonyldiimidazol werden nach der in Beispiel 1 beschriebenen Weist 185 mg 4-[2-(N-Butyl-N-methylamino)-ethyl]-2,3-dihydro-2-benzimidazolon, Hydrobromid vom Schmelzpunkt 240 - 242 °C erhalten.

### Beispiel 7

### 4-[2-[N-(2-Phenylethyl)-N-propylamino]-ethyl]-2,3-dihydro-2-benzimidazolon, Fumarat

Aus 250 mg N-[2-(2,3-Diaminophenyl)-ethyl]-N-(2-phenylethyl)-N-propylamin und 250 mg N,N′-Carbonyldiimidazol werden nach der in Beispiel3 beschriebenen Weise 147 mg 4-[2-[N-(2-Phenylethyl)-N-propylamino]-ethyl]-2,3-dihydro-2-benzimidazolon, Fumarat vom Schmelzpunkt 176-177 °C erhalten.

### Beispiel 8

### 4-[2-[N-Propyl-N-[2-(2-thienyl)-ethylamino]]-ethyl]-2,3-dihydro-2-benzimidazolthion, Hydrobromid

Aus 450 mg N-[2-(2,3-Diaminophenyl)-ethyl]-N-[2-(2-thienyl)-ethyl]-N-propylamin und 400 mg N,N′-Thiocarbonyldiimidazol werden nach der in Beispiel 1 beschriebenen Weise 260 mg 4-[2-[N-Propyl-N-[2-(2-thienyl)-ethylamino]]-ethyl]-2,3-dihydro-2-benzimidazolthion, Hydrobromid vom Schmelzpunkt 193-195 °C erhalten.

### Beispiel 9

### 4-[2-[N-Propyl-N-[2-(3-thienyl)-ethylamino]]-ethyl]-2,3-dihydro-2-benzimidazolon, Fumarat

Aus 450 mg N-[2-(2,3-Diaminophenyl)-ethyl]-N-[2-(3-thienyl)-ethyl]-N-propylamin und 230 mg N,N′-Carbonyldiimidazol werden nach der in Beispiel 3 beschriebenen Weise 173 mg 4-[2-[N-Propyl-N-[2-(3-thienyl)-ethylamino]]-ethyl]-2,3-dihydro-2-benzimidazolon, Fumarat vom Schmelzpunkt 114-117°C erhalten.

### Beispiel 10

### 4-[2-[N-Propyl-N-[2-(3-thienyl)-ethylamino]]-ethyl]-2,3-dihydro-2-benzimidazolthion, Fumarat

Aus 900 mg N-[2-(2,3-Diaminophenyl)-ethyl]-N-[2-(3-thienyl)-ethyl]-N-propylamin und 500 mg N,N′-Thiocarbonyldiimidazol werden nach der in Beispiel 3 beschriebenen Weise 455 mg 4-[2-[N-Propyl-N-[2-(3-thienyl)-ethylamino]]-ethyl]-2,3-dihydro-2-benzimidazolthion, Fumarat vom Schmelzpunkt 207-209°C erhalten.

## Patentansprüche

1. Benzimidazol-Derivate der allgemeinen Formel I 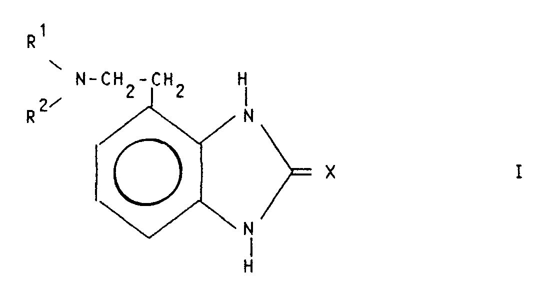 worin
R¹ und R² gleich oder verschieden sind und jeweils Wasserstoff, einen Cycloalkyl- oder Cycloalkylalkylrest mit 3 - 6 Kohlenstoffatomen oder einen Alkylrest mit 1 - 6 Kohlenstoffatomen bedeuten, wobei der Alkylrest ein-oder zweifach mit Phenyl oder einem 5 - oder 6-Ring-Heteroaromaten mit ein bis zwei Schwefel-, Stickstoff- und/oder Sauerstoffatomen substituiert sein kann und der Phenylrest ein- bis dreifach mit C₁₋₄-Alkyl, C₁₋₄-Alkoxy, Hydroxy oder Halogen substituiert sein kann und
X Sauerstoff oder Schwefel ist, sowie deren Säureadditionssalze oder Tautomere.

2. 4-[2-(N,N-Dipropylamino)-ethyl]-2,3-dihydro-2-benzimidazolthion
4-(2-(N,N-Dipropylamino)-ethyl]-2,3-dihydro-2-benzimidazolon
4-[2-N-Butyl-N-methylamino)-ethyl]-2,3-dihydro-2-benzimidazolon
4-[2-[N-(2-Phenylethyl)-N-propylamino]-ethyl]-2,3-dihydro-2-benzimidazolon
4-[2-[N-Propyl-N-[2-(2-thienyl)-ethylamino]ethyl]-2,3-dihydro-2-benzimidazolthion
4[2-(N-Butyl-N-methylamino)-ethyl]-2,3-dihydro-2-benzimidazolthion
4-[2-[N-(2-Phenylethyl)-N-propylamino]-ethyl]-2,3-dihydro-2-benzimidazolthion
4-[2-[N-Propyl-N-[2-(2-thienyl)-ethylamino]]-ethyl]-2,3-dihydro-2-benzimidazolon
4-[2-[N-Propyl-N-[2-(3-thienyl)-ethylamino]]-ethyl]-2,3-dihydro-2-benzimidazolon
4-[2-[N-Propyl-N-[2-(3-thienyl)-ethylamino]]-ethyl-2,3-dihydro-2-benzimidazolthion

3. Verfahren zur Herstellung der Verbindungen der allgemeinen Formel I, indem man Verbindungen der allgemeinen Formel II 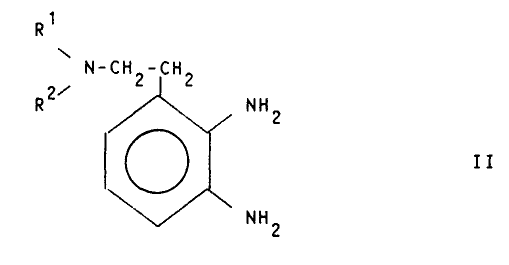 worin
R¹ und R² die obige Bedeutung haben mit einem Kohlensäure- oder Thiokohlensäurederivat cyclisiert und gewünschtenfalls anschließend die Säureadditionssalze bildet.

4. Arzneimittel auf Basis der Verbindungen gemäß Anspruch 1 und 2.

5. Verwendung von Verbindungen gemäß den Ansprüchen 1 und 2 zur Herstellung von Arzneimitteln.

6. Verfahren zur Herstellung eines Arzneimittels, dadurch daß man eine nach Anspruch 3 hergestellte Verbindung der Formel I mit einem pharmazeutischen Träger mischt.

## Claims

1. Benzimidazole derivatives of the general formula I 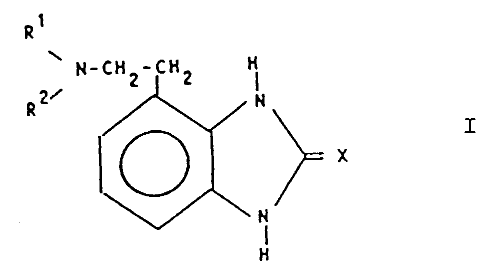 wherein
R¹ and R² are identical or different and are each hydrogen, a cycloalkyl or cycloalkylalkyl radical having from 3 to 6 carbon atoms or an alkyl radical having from 1 to 6 carbon atoms, wherein the alkyl radical may be mono- or di-substituted by phenyl or by a 5- or 6-membered-ring heteroaromatic radical having one or two sulphur, nitrogen and/or oxygen atoms, and the phenyl radical may be mono- to tri-substituted by C₁₋₄alkyl, C₁₋₄alkoxy, hydroxy or by halogen, and
x is oxygen or sulphur,
and the acid addition salts or tautomeric forms thereof.

2. 4-[2-(N,N-dipropylamino)-ethyl]-2,3-dihydro-2-benzimidazolethione,
4-[2-(N,N-dipropylamino)-ethyl]-2,3-dihydro-2-benzimidazolone,
4-[2-(N-butyl-N-methylamino)-ethyl]-2,3-dihydro-2-benzimidazolone,
4-[2-[N-(2-phenylethyl)-N-propylamino]-ethyl]-2,3-dihydro-2-benzimidazolone,
4-[2-[N-propyl-N-[2-(2-thienyl)-ethyl]-amino]-ethyl]-2,3-dihydro-2-benzimidazolethione,
4-[2-(N-butyl-N-methylamino)-ethyl]-2,3-dihydro-2-benzimidazolethione,
4-[2-[N-(2-phenylethyl)-N-propylamino]-ethyl]-2,3-dihydro-2-benzimidazolethione,
4-[2-[N-propyl-N-[2-(2-thienyl)-ethyl]-amino]-ethyl]-2,3-dihydro-2-benzimidazolone,
4-[2-[N-propyl-N-[2-(3-thienyl)-ethyl]-amino]-ethyl]-2,3-dihydro-2-benzimidazolone,
4-[2-[N-propyl-N-[2-(3-thienyl)-ethyl]-amino]-ethyl]-2,3-dihydro-2-benzimidazolethione.

3. A process for the preparation of the compounds of the general formula I, by cyclising compounds of the general formula II 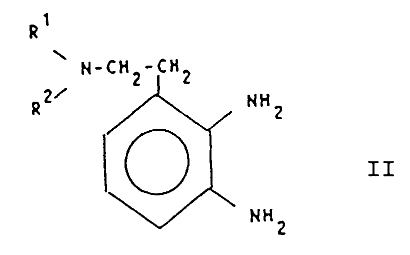 wherein R¹ and R² have the meanings given above, with a carbonic acid or thiocarbonic acid derivative and, if desired, subsequently forming the acid addition salts.

4. Medicaments based on compounds according to claims 1 and 2.

5. The use of compounds according to claims 1 and 2 for the preparation of medicaments.

6. Process for the preparation of a medicament, by mixing a compound of the formula I prepared according to claim 3 with a pharmaceutical carrier.

## Revendications

1. Dérivés du benzimidazole qui répondent à la formule générale I : 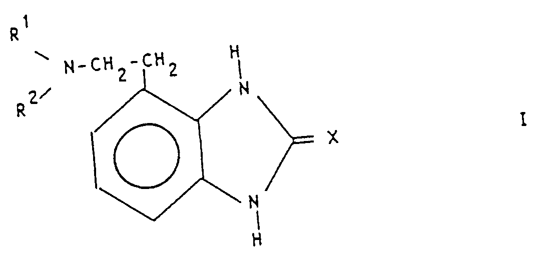 dans laquelle :
R¹ et R² représentent chacun, indépendamment l'un de l'autre, l'hydrogène, un radical cycloalkyle ou cycloalkyl-alkyle contenant de 3 à 6 atomes de carbone ou un radical alkyle contenant de 1 à 6 atomes de carbone, le radical alkyle pouvant porter un ou deux substituants pris dans l'ensemble constitué par phenyle et les hétéroaryles à cycle pentagonal ou hexagonal qui contiennent un ou deux atomes de soufre, d'azote et/ou d'oxygène, et le radical phenyle pouvant porter de un à trois substituants pris dans l'ensemble constitué par les alkyles en C₁-C₄, les alcoxy en C₁-C₄, l'hydroxy et les halogènes, et
X représente l'oxygène ou le soufre, ainsi que leurs sels d'addition d'acides ou leurs formes tautomeres.

2. 4-[2(N,N-Dipropylamino)-éthyl]-2,3-dihydro-2-benzimidazole-thione,
4-[2-(N,N-dipropylamino)-éthyl]-2,3-dihydro-2-benzimidazolone,
4-[2-(N-butyl-N-méthylamino)-éthyl]-2,3-dihydro-2-benzimidazolone,
4-[2-(N-(2-phényléthyl)-N-propylamino]-éthyl]-2, 3-dihydro-2-benzimidazolone,
4-[2-[N-propyl-N-[2-(2-thiényl)-éthyl]-amino]-éthyl]-2,3-dihydro-2-benzimidazole-thione,
4-[2-(N-butyl-N-méthylamino)-éthyl]-2,3-dihydro-2-benzimidazole-thione,
4-[2-[N-(2-phényléthyl)-N-propylamino]-éthyl]-2, 3-dihydro-2-benzimidazole-thione,
4-[2-[N-propyl-N-[2-(2-thiényl)-éthyl]-amino]-éthyl]-2,3-dihydro-2-benzimidazolone,
4-[2-[N-propyl-N-[2-(3-thiényl)-éthyl]-amino]-étnyl] -2,3-dihydro-2-benzimidazolone et
4-[2-[N-propyl-N-[2-(3-thiényl)-éthyl] amino]-éthyl]-2,3-dihydro-2-benzimidazole-thione.

3. Procédé pour préparer les composés de formule générale I, procédé selon lequel on cyclise des composés répondant à la formule générale II : 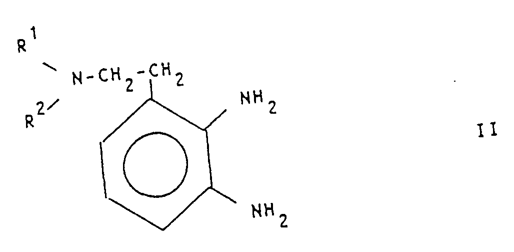 dans laquelle R¹ et R² ont les significations qui leur ont été données ci-dessus, avec un dérivé de l'acide carbonique ou de l'acide thiocarbonique, puis, si on le désire, on forme les sels d'addition d'acides.

4. Médicaments à base de composés selon l'une des revendications 1 et 2.

5. Application de composés selon l'une des revendications 1 et 2 pour la fabrication de médicaments.

6. Procédé pour fabriquer un médicament caractérisé en ce qu'on mélange un composé de formule I préparé selon la revendication 3 avec un excipient pharmaceutique.
